(19) Europäisches Patentamt
European Patent Office
Office européen des brevets

(11) Publication number: **0 041 246**
**B1**

(12) # EUROPEAN PATENT SPECIFICATION

(45) Date of publication of patent specification: **13.07.83**

(51) Int. Cl.³: **C 07 D 285/16**, C 07 F 9/65 //A01N57/16

(21) Application number: **81104113.6**

(22) Date of filing: **05.03.79**

(60) Publication number of the earlier application in accordance with Art. 76 EPC: **0018368**

(54) **2-Substituted-imino-3-alkyl-tetrahydro-6H-1,3,4-thiadiazine-5-ones salts thereof and their preparation.**

(30) Priority: **24.03.78 US 889757**
**24.03.78 US 889758**

(43) Date of publication of application:
**09.12.81 Bulletin 81/49**

(45) Publication of the grant of the patent:
**13.07.83 Bulletin 83/28**

(84) Designated Contracting States:
**FR**

(56) References cited:
**None**

(73) Proprietor: **CHEVRON RESEARCH COMPANY**
**P.O. Box 7643**
**San Francisco, CA 94120 (US)**

(72) Inventor: **Edwards, Laroy H.**
**1765 Silverado Trail**
**Napa California 94558 (US)**
Inventor: **Cleveland, James D.**
**724 Cerrito Street**
**Albany California 94706 (US)**

(74) Representative: **Rinuy, Guy et al,**
**14, Avenue de la Grande Armée**
**F-75017 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 041 246 B1

2-substituted-imino-3-alkyl-tetrahydro-6H-1,3,4-thiadiazine-5-ones, salts thereof and their preparation

The present invention relates to thiadiazine compounds, their preparation and their use for preparing valuable insecticides.

Chem. Abstr., Vol. 80, 82906p (1974), discloses thiadiazinones and their mono- and dithiophosphates.

The novel compounds of the invention are 2-(substituted-imino)-3-alkyl-tetrahydro-6H-1,3,4-thiadiazin-5-ones represented by the formula

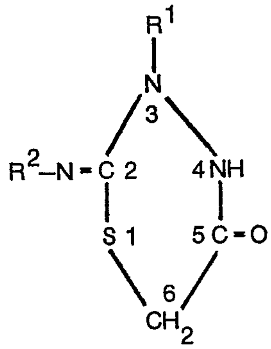

(II)

wherein $R^1$ is alkyl of 1 to 6 carbon atoms and, $R^2$ is alkyl of 1 to 6 carbon atoms, alkenyl of 3 to 6 carbon atoms, cycloalkyl of 5 to 6 carbon atoms, phenyl, or phenyl substituted with 1 to 2 substituents selected from the group consisting of alkyl of 1 to 6 carbon atoms, fluoro, chloro, bromo, iodo, nitro, trifluoromethyl, trichloromethyl, tribromomethyl and alkoxy of 1 to 4 carbon atoms.

Representative alkyl $R^1$ and $R^2$ groups include methyl, ethyl, isopropyl, n-butyl, isohexyl, n-hexyl, etc.

Representative substituted phenyl $R^2$ groups include 4-methylphenyl, 2,4-dimethylphenyl, 2-fluorophenyl, 3,5-dichlorophenyl, 3-bromophenyl, 4-iodophenyl, 2-methyl-4-chlorophenyl, 4-nitrophenyl, 3-trichloromethylphenyl, 2-methoxyphenyl and 2-chloro-4-nitrophenyl. Representative alkenyl $R^2$ groups are allyl, 2-butenyl and 3-hexenyl.

Preferably $R^1$ is lower alkyl of 1 to 3 carbon atoms, especially methyl.

Preferred alkyl $R^2$ groups are alkyl of 1 to 4 carbon atoms and preferred aryl $R^2$ groups are phenyl and phenyl substituted with 1 to 2 substituents selected from alkyl of 1 to 4 carbon atoms, fluoro, chloro, bromo, trifluoromethyl and trichloromethyl. Preferably, $R^2$ is alkyl of 1 to 4 carbon atoms.

A preferred class of thiadiazines of formula (II) is that wherein $R^1$ is lower alkyl of 1 to 3 carbon atoms, especially methyl and $R^2$ is alkyl of 1 to 6 carbon atoms.

The novel compounds according to the present invention can be prepared by the cyclization of a semithiocarbazide (IV) with a haloacetic anhydride (V), as depicted in the following reaction (2):

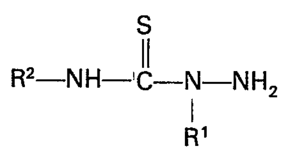 + $(X\ CH_2CO)_2O$ → (II) (2)

(IV) (V)

wherein $R^1$ and $R^2$ have the same meaning as previously stated, and X is chloro or bromo, preferably chloro.

Reaction (2) is generally conducted by reacting substantially equimolar amounts of the semithiocarbazide (IV) and the haloacetic anhydride (V) in the liquid phase. The molar ratios of semithiocarbazide to anhydride (V) generally vary from about 1:1.2 to 1.2:1, although molar ratios of from about 1:1.1 to 1.1:1 are preferred. The reaction is normally conducted in an inert liquid diluent, e.g., organic solvents such as chlorinated hydrocarbons. The reaction is conducted at a temperature of from about 0° to the boiling point of the diluent, although temperatures from about 25°C to 100°C are preferred. The reaction is generally conducted at or above atmospheric pressure. Generally, the reaction is completed within one-half to 24 hours. The product (II) can be isolated and purified by conventional procedures such as extraction, filtration, crystallization and chromatography. Generally, when the thiadiazinone product (II) has an aliphatic imino substituent ($R^2$), the product is most conveniently isolated as a hydrogen halide salt.

The thiadiazin-5-one compounds (II) can also be prepared by reacting the semithiocarbazide (IV) with an alkyl halothioacetate (VI) as depicted in the following reaction (3):

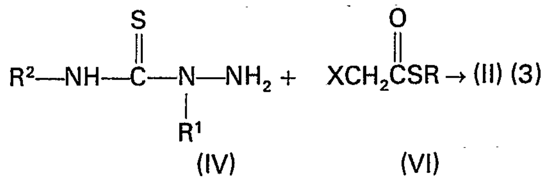

(IV) (VI)

wherein $R^1$, $R^2$ and X have the same meaning as previously stated and R is alkyl of 1 to 6 carbon atoms, preferably of 1 to 3 carbon atoms.

Reaction (3) is generally conducted by reacting substantially equimolar amounts of the semithiocarbazide (1V) and the halothioacetate (VI) in the liquid phase. The molar ratios of semithiocarbazide to halothioacetate (VI) generally vary from about 1:1.2 to 1.2:1, although molar ratios of from about 1:1.1 to 1.1:1 are preferred. The reaction is normally conducted in an inert liquid diluent. Suitable inert organic diluents include alkanones such as acetone, methyl ethyl ketone; acyclic alkyl ethers such as dimethoxyethane and dibutylether; cyclic ethers such as dixoane or tetrahydrofuran; haloalkanes such as dichloromethane; and aromatic compounds such as benzene, toluene, and chlorobenzene. Generally, the amount of diluent employed ranges from 1 to 50 mols per mol of semithiocarbazide (IV). The reaction can be conducted at a temperature of from about 0°C to the boiling point of the diluent, although temperatures from about 25°C to 100°C are preferred. The reaction is conducted at or above atmospheric pressure. Generally, the reaction is completed within one-half to 24 hours. The product (II) can be isolated and purified by conventional procedures such as extraction, filtration, crystallization and chromatography. Generally, when the thiadiazinone product (II) has an aliphatic imino substituent ($R^2$), the product is most conveniently isolated as a hydrogen halide salt.

The compounds of the present invention of formula (II) may be used for preparing by the addition of a dialkoxyhalothiophosphate (III), a 2-(substituted-imino)-3-alkyl-5-dialkoxy-phosphinothioloxy-6H-1,3,4-thiadiazine (I), as depicted in the following reaction (1)

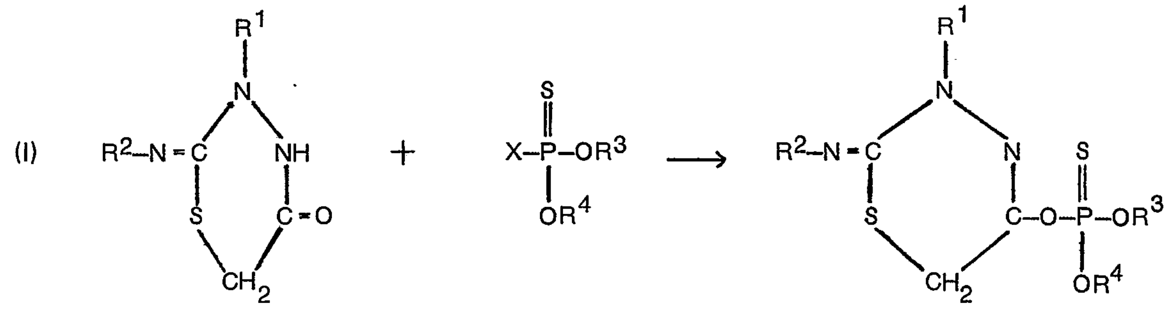

(II) (III) (I)

wherein $R^1$ and $R^2$ and X have the same meaning as stated before, $R^3$ is alkyl of 1 to 6 carbon atoms and $R^4$ is alkyl of 1 to 6 carbon atoms.

The said compounds of formula (I) are described and claimed in the parent European patent application 79.900348.8 published under publication No 18,368.

Reaction (1) can generally be conducted by reacting substantially equimolar amounts of the thiadiazinone (II) and the thiophosphate (III), i.e., the molar ratios of thiadiazinone (II) to thiophosphate (III) generally vary from about 1:1.2 to 1.2:1, although molar ratios from about 1:1.1 to 1.1:1 are preferred. A substantially equivalent amount of a base can be used to scavenge the hydrogen halide byproduct. Such bases are preferably inorganic bases, e.g., alkali metal carbonates such as sodium carbonate and potassium carbonate, and alkali metal bicarbonates such as sodium bicarbonate. The molar ratio of base to the thiadiazinone (II) is generally about 1.1:1 to 1:1. The reaction can be conducted in an inert liquid organic diluent. Suitable inert organic diluents include alkanones such as acetone, methyl ethyl ketone; acyclic alkyl ethers such as dimethyloxyethane and dibutylether; cyclic ethers such as dioxane or tetrahydrofuran; haloalkanes such as dichloromethane and aromatic compounds such as benzene, toluene, and chlorobenzene. Generally, the amount of diluent employed ranges from 1 to 50 mols per mol thiophosphate (III).

Reaction (1) is suitably conducted at a temperature of from about 15°C to the boiling point of the diluent, although temperatures of from about 20°C to 100°C are preferred. The reaction is generally conducted at or above atmospheric pressure. The reaction time will, of course, vary depending on the reaction temperature and the particular reactants employed. Generally, however, the reacton time

varies from several minutes to 24 hours. The thiadiazine product (I) can be isolated from the reaction mixture by conventional procedures, e.g., extraction, chromatography, crystallization, etc.

Reaction (1) can also be conducted with the hydrogen halide salts of the thiadiazinone (II). In this modification of reaction (1), two equivalents of the base material are generally used, i.e. the molar ratio of base to thiadiazinone is generally about 2.2:1 to 2:1. It is generally preferred to use the hydrogen halide salt when the imino substituent ($R^2$) of the thiadiazinone (II) is aliphatic. The compounds of formula (I) are valuable insecticides.

Example 1

Preparation of 2-(3,4-dichlorophenylimino)-3-methyl-tetrahydro-6H-1,3,4-thiadiazin-5-one

To a stirred solution of 37.5 g (0.15 mol) 2-methyl-4-(3,4-dichlorophenyl)semithiocarbazide in 200 ml dichloromethane was added 25.5 g (0.015 mol) chloroacetic anhydride. The resulting reaction mixture was heated under reflux for 16 hours, cooled, neutralized with sodium bicarbonate, washed with water, dried over magnesium sulfate and evaporated under reduced pressure to give an oily solid. The solid was slurried with ether, filtered and dried to give 12 g of the product, as a grey solid, m.p. 118°—120°C. This product is tabulated in Table A as compound No. A—6.

Example 2

Preparation of 2-(t-butylimino)-3-methyl-tetrahydro-6H-1,3,4-thiadiazin-5-one hydrochloride

To a stirred solution of 16.1 g (0.1 mol) 2-methyl-4-t-butylsemithiocarbazide in 200 ml dichloromethane was added slowly 17.1 g (0.1 mol) chloroacetic anhydride. The resulting solution was stirred at about 25°C. for about 8 hours. The reaction mixture was filtered to give 14 g of the product as a white solid, m.p., 224°—225°C. The infrared spectrum of the product showed carbonyl absorption at 5.95 micron. Elemental analysis for $C_8H_{16}Cl\,N_3OS$ showed:

| | Calc, | Found |
|---|---|---|
| %S | 13.5 | 13.3 |
| %Cl | 14.9 | 15.5 |

Example 3

Preparation of 2-(t-butylimino-3-methyl-tetrahydro-6H-1,3,4-thiadiazin-5-one hydrobromide

To a stirred solution of 5.75 g (0.05 mol) 2-methyl-4-t-butylsemithiocarbazide in 50 ml dichloromethane was added dropwise 9.15 g (0.05 mol) ethyl alpha-bromothioacetate at 25°C. The reaction mixture was stirred at 25°C for 20 minutes (slightly exothermic reaction) and then heated under reflux for 4 hours. After cooling, the precipitated solid was filtered off, washed with hexane and dried to give 7.3 g of product, m.p. 215—216°C.

Example 4

Preparation of 2 - (3,4 - dichlorophenylimino - 3 - methyl - 5 - diethoxyphosphinothioyloxy) - 2,3 - dihydro - 6H - 1,3,4 - thiadiazine

A slurry of 14 g (0.048 mol) 2 - (3,4 - dichlorophenylimino) - 3 - methyl - tetrahydro - 6H - 1,3,4 - thiadiazin - 5 - one, 7 g (0.048 mol) patassium bicarbonate, and 9.1 g (0.048 mol) diethylchlorothiophosphate in 200 ml acetone was heated under reflux for 8 hours, cooled and stirred at about 25°C for overnight. The reaction mixture was filtered and evaporated under reduced pressure to give an oil. The oil was chromatographed on silica gel using 50/50 petroleum ethyl/diethyl ether as the eluant to give 11 g of the product, as a yellow oil. This product is tabulated in Table B, as compound B—7.

Example 5

Preparation of 2 - (t - butylimino) - 3 - methyl - 5 - diethoxyphosphinothioyloxy - 2,3 - dihydro-6H - 1,3,4 - thiadiazine

A slurry of 10.0 g (0.0421 mol) 2-(t-butylimino)-3-methyl-6H-1,3,4-thiadiazin-5-one, 11.6 g (0.0842 mol) potassium carbonate and 8.2 g (0.0421 mol) diethylchlorothiophosphate in 200 ml acetone was heated under reflux for 8 hours. The reaction mixture cooled, stirred overnight, filtered and evaporated under reduced pressure to give an oil residue. The residue was dissolved in dichloromethane, washed with water, dried over magnesium sulfate and evaporated under reduced pressure. The crude product was then chromatographed on silica gel using diethyl ether as the eluant. The purified product (6 g) was an amber oil. The product is tabulated in Table B as Compound No. B—1.

The compounds tabulated in Tables A and B were prepared by procedures similar to those of Examples 1—5. The structure of each compound tabulated in Tables A and B was confirmed by nuclear magnetic resonance and/or infrared spectroscopy.

TABLE A

Compounds of the formula

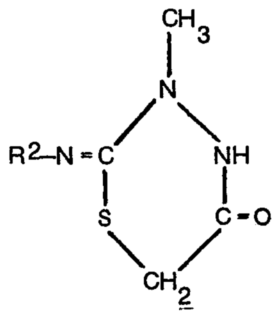

| No. | $R^2$ | m.p.,°C | Elemental Analysis C Calc. | C Found | H Calc. | H Found | N Calc. | N Found |
|---|---|---|---|---|---|---|---|---|
| A—1 | 4-Cl-$\phi$ | 143—145 | — | — | — | — | 12.5[1] | 13.2[1] |
| A—2 | i-$C_3H_7$ | 122—124 | — | — | — | — | 14.1[1] | 13.8[1] |
| A—3 | n-$C_3H_7$ | 126—130 | 37.6 | 35.6 | 6.3 | 5.9 | 18.2 | 17.2 |
| A—4 | t-$C_4H_9$ | 127—129 | 40.4 | 39.3 | 6.8 | 6.6 | 17.7 | 17.1 |
| A—5 | 3-$CF_3$-$\phi$ | 83—84 | 45.7 | 44.4 | 3.5 | 4.1 | 14.5 | 15.0 |
| A—6 | 3,4-$Cl_2$-$\phi$ | 118—120 | 41.4 | 40.7 | 3.1 | 3.1 | 14.5 | 14.1 |
| A—8 | cyclohexyl | 133—135 | 45.6 | 47.1 | 6.9 | 7.8 | 15.9 | 16.8 |
| A—9 | $CH_2$=$CHCH_2$ | 68—70 | 37.9 | 35.1 | 5.5 | 5.6 | 19.0 | 17.9 |
| A—10 | $\phi$ | 143—145 | 54.3 | 55.1 | 5.0 | 5.2 | 19.0 | 19.4 |

[1] Sulfur analysis

$\phi$ represents phenyl.

TABLE B

Compounds of the formula

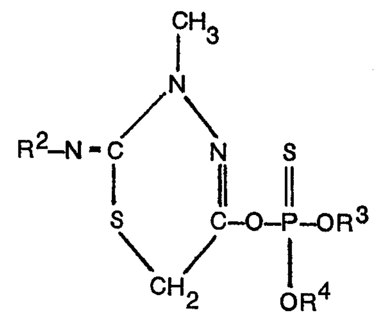

| No. | $R^2$ | $R^3$ | $R^4$ | m.p., °C | Elemental Analysis C Calc. | C Found | H Calc. | H Found | N Calc. | N Found |
|---|---|---|---|---|---|---|---|---|---|---|
| B—1 | t-$C_4H_9$ | $C_2H_5$ | $C_2H_5$ | oil | — | — | — | — | 18.1[1] | 17.9[1] |
| B—2 | n-$C_3H_7$ | $C_2H_5$ | $C_2H_5$ | oil | — | — | — | — | 18.9[1] | 19.4[1] |
| B—4 | cyclohexyl | $C_2H_5$ | $C_2H_5$ | oil | 44.3 | 43.6 | 6.9 | 6.9 | 11.1 | 10.8 |
| B—6 | i-$C_3H_7$ | $C_2H_5$ | $C_2H_5$ | oil | 39.9 | 38.4 | 6.5 | 6.4 | 12.4 | 11.4 |
| B—7 | 3,4-$Cl_2$-$\phi$ | $C_2H_5$ | $C_2H_5$ | oil | 38.0 | 37.4 | 4.1 | 4.3 | 9.5 | 9.7 |
| B—9 | $CH_2$=$CHCH_2$ | $C_2H_5$ | $C_2H_5$ | oil | 39.2 | 37.1 | 6.0 | 5.7 | 12.5 | 11.5 |
| B—10 | $\phi$ | $C_2H_5$ | $C_2H_5$ | oil | 45.0 | 41.4 | 5.4 | 5.4 | 11.3 | 10.0 |
| B—11 | $CH_2$=$CHCH_2$ | $CH_3$ | $CH_3$ | oil | 35.0 | 36.7 | 5.2 | 5.2 | 13.6 | 13.7 |
| B—12 | i-$C_3H_7$ | $CH_3$ | $CH_3$ | oil | 34.7 | 36.1 | 5.8 | 6.0 | 13.5 | 13.5 |

[1] Sulfur analysis

$\phi$ represents phenyl.

**Claims**

1. A compound of the formula

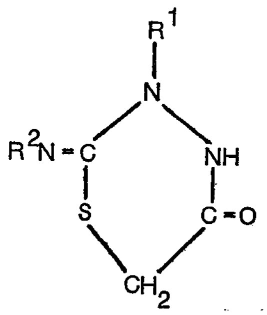

wherein $R^1$ is alkyl of 1 to 6 carbon atoms and $R^2$ is alkyl of 1 to 6 carbon atoms, alkenyl of 3 to 6 carbon atoms, cycloalkyl of 5 to 6 carbon atoms, phenyl, or phenyl substituted with 1 to 2 substituents selected from the group consisting of alkyl of 1 to 4 carbon atoms, fluoro, chloro, bromo, iodo, nitro, trifluoromethyl, trichloromethyl, tribromomethyl and alkoxy of 1 to 4 carbon atoms.

2. A compound according to Claim 1, in which $R^1$ is alkyl of 1 to 3 carbon atoms.

3. A compound according to Claim 1 or 2 in which $R^2$ is alkyl of 1 to 6 carbon atoms.

4. A compound according to any preceding Claim in which $R^1$ is methyl and $R^2$ is t-butyl.

5. A method of preparing a compound according to Claim 1 which comprises reacting a thiosemicarbamide of the formula

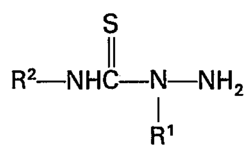

wherein $R^1$ and $R^2$ are as defined in Claim 1, with chloroacetic or bromoacetic anhydride in the liquid phase.

6. A method of preparing a compound according to Claim 1, which comprises reacting a thiosemicarbaside of the formula

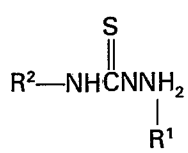

wherein $R^1$ and $R^2$ are as defined in Claim 1, with an alkyl alphahalothioacetate wherein said alkyl has 1 to 6 carbon atoms and the halo is chloro or bromo in the liquid phase.

7. A method according to the Claim 6, wherein said alkyl has 1 to 3 carbon atoms.

8. A method according to Claim 6 or 7, wherein $R^2$ is alkyl and said compound is isolated as a hydrogen halide salt.

9. A method according to any one of Claims 6 to 8, wherein the reaction temperature is from about 25°C to 100°C.

10. A method according to any one of Claims 6 to 9, wherein the molar ratio of thiosemicarbazide to thioacetate is about 1:1.2 to 1.2:1.

**Revendications**

1. Composé de formule

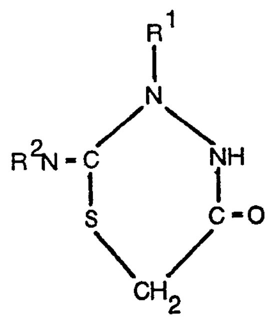

dans laquelle $R^1$ est un groupe alkyle ayant 1 à 6 atomes de carbone et $R^2$ est un groupe alkyle ayant 1 à 6 atomes de carbone, alcényle ayant 3 à 6 atomes de carbone, cycloalkyle ayant 5 ou 6 atomes de carbone, phényl ou phényle substitué avec 1 ou 2 substituants choisis dans le groupe formé des radicaux alkyle ayant 1 à 4 atomes de carbone, fluoro, chloro, bromo, iodo, nitro, trifluorométhyle, trichlorométhyle, tribromométhyle et alkoxy ayant 1 à 4 atomes de carbone.

2. Composé suivant la revendication 1, dans lequel $R^1$ est un groupe alkyle ayant 1 à 3 atomes de carbone.

3. Composé suivant la revendication 1 ou 2, dans lequel $R^2$ est un groupe alkyle ayant 1 à 6 atomes de carbone.

4. Composé suivant l'une quelconque des revendications précédentes, dans lequel $R^1$ est un groupe méthyle et $R^2$ est un groupe tertio-butyle.

5. Procédé de préparation d'un composé suivant la revendication 1, qui consiste à faire réagir un thiosemicarbazide formule

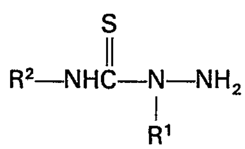

dans laquelle $R^1$ et $R^2$ ont les définitions données dans la revendication 1, avec l'anhydride chloracétique ou bromacétique en phase liquide.

6. Procédé de préparation d'un composé suivant la revendication 1, qui consiste à faire réagir un thiosemicarbazide de formule

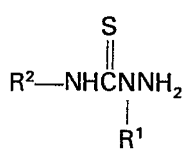

dans laquelle $R^1$ et $R^2$ ont les définitions données dans la revendication 1, avec un alpha-halogénothioacétate d'alkyle dont le groupe alkyle comprend 1 à 6 atomes de carbone et le radical halogéno est le radical chloro ou bromo, en phase liquide.

7. Procédé suivant la revendication 6, dans lequel le groupe alkyle comprend 1 à 3 atomes de carbone.

8. Procédé suivant la revendication 6 ou 7, dans lequel $R^2$ est un groupe alkyle et ledit composé est isolé sous la forme d'un halogénhydrate.

9. Procédé suivant l'un quelconque des revendications 6 à 8, dans lequel la température de réaction va d'environ 25 à 100°C.

10. Procédé suivant l'une quelconque des revendications 6 à 9, dans lequel le rapport molaire du thiosemicarbazide au thio-acétate est d'environ 1:1,2 à 1,2:1.

**Patentansprüche**

1. Eine Verbindung der Formel

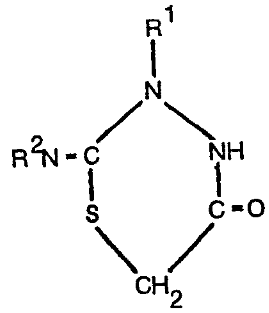

worin $R^1$ ein Alkyl mit 1 bis 6 Kohlenstoffatomen und $R^2$ ein Alkyl mit 1 bis 6 Kohlenstoffatomen, Alkenyl mit 3 bis 6 Kohlenstoffatomen, Cycloalkyl mit 5 bis 6 Kohlenstoffatomen, Phenyl oder ein substituiertes Phenyl mit 1 bis 2 Substituenten, die ausgewählt sind aus der Gruppe Alkyl mit 1 bis 4 Kohlenstoffatomen, Fluor, Chlor, Brom, Iod, Nitro, Trifluormethyl, Trichlormethyl, Tribrommethyl und Alkoxy mit 1 bis 4 Kohlenstoffatomen, ist.

2. Verbindung nach Anspruch 1, worin $R^1$ ein Alkyl mit 1 bis 3 Kohlenstoffatomen ist.

3. Verbindung nach Anspruch 1 oder 2, worin $R^2$ ein Alkyl mit 1 bis 6 Kohlenstoffatomen ist.

4. Verbindung nach einem der vorangehenden Ansprüche, worin $R^1$ Methyl und $R^2$ t-Butyl ist.

5. Verfahren zum Herstellen einer Verbindung nach Anspruch 1, wobei ein Semicarbazid der Formel

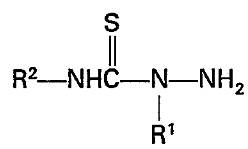

worin $R^1$ und $R^2$ die in Anspruch 1 angegebenen Bedeutungen haben, mit Chloracetanhydrid oder Bromacetanhydrid in der flüssigen Phase umgesetzt wird.

6. Verfahren zum Herstellen einer Verbindung nach Anspruch 1, wobei ein Thiosemicarbazid der Formel

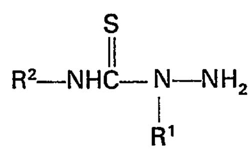

worin $R^1$ und $R^2$ die in Anspruch 1 angegebenen Bedeutungen haben, mit einem Alkylalphahalothioacetat, worin das Alkyl 1 bis 6 Kohlenstoffatome hat und das Halogen Chlor oder Brom ist, in der flüssigen Phase umgesetzt wird.

7. Verfahren nach Anspruch 6, worin das Alkyl 1 bis 3 Kohlenstoffatome hat.

8. Verfahren nach Anspruch 6 oder 7, worin $R^2$ Alkyl ist und die Verbindung als ein Halogenwasserstoffsalz isoliert wird.

9. Verfahren nach einem der Ansprüche 6 bis 8, worin die Reaktionstemperatur von etwa 25°C bis 100°C ist.

10. Verfahren nach einem der Ansprüche 6 bis 9, worin das Molverhältnis von Thiosemicarbazid zu Thioacetat etwa 1:1,2 bis 1,2:1 ist.